# EUROPEAN PATENT APPLICATION

(11) **EP 4 736 934 A1**
(43) Date of publication of application: **06.05.2026**
(21) Application number: 24830554.2
(22) Date of filing: 18.06.2024
(51) Int. Cl.: A61N 1/36, A61N 1/34, A61N 1/05

(54) **PULSE GENERATOR, STIMULATOR, STORAGE MEDIUM, AND PROGRAM PRODUCT**

(30) Priority: 27.06.2023 CN 202310760165
(71) Applicant: Sceneray Co., Ltd, Suzhou, Jiangsu 215000 (CN)
(72) Inventor: ZHI, Menghui, Suzhou, Jiangsu 215000 (CN); ZHU, Weiran, Suzhou, Jiangsu 215000 (CN); LU, Haiyang, Suzhou, Jiangsu 215000 (CN)
(74) Representative: Cabinet Beaumont
(86) International application number: PCT/CN2024/099711
(87) International publication number: WO 2025/001908

(57) **Abstract**

Provided are a pulse generator, a stimulator, a computer-readable storage medium and computer program product. At least one processor of the pulse generator is configured to, when executing a computer program, implement the following steps: acquiring power spectral density amplitudes of bioelectrical signals corresponding to each contact delivering electrical stimulation to internal tissue of the patient and each contact not delivering electrical stimulation, and recording the power spectral density amplitudes as a first density amplitude and a second density amplitude, respectively; determining one contact among multiple contacts as a target contact according to the first density amplitude and the second density amplitude corresponding to each contact; acquiring multiple candidate contact pairs near the target contact; acquiring a power spectral density amplitude of a bioelectric signal corresponding to each candidate contact pair, and recording the power spectral density amplitude as a third density amplitude; and determining a target acquisition contact pair.

## Description

This application claims priority to Chinese Patent Application No. 202310760165.8 filed with the China National Intellectual Property Administration on June 27, 2023, the disclosure of which is incorporated herein by reference in its entirety.

### TECHNICAL FIELD

The present disclosure relates to the technical field of implantable medical devices, for example, to a pulse generator, a stimulator, a computer-readable storage medium and a computer program product.

### BACKGROUND

In the related art, deep brain stimulation (DBS) treatment involves delivering electrical stimulation to a neural structure in a specific region of the brain to excite or inhibit cell activity, which can effectively treat conditions, such as chronic pain, movement disorders like Parkinson's disease and essential tremor, and epilepsy, and mental illnesses, such as depression and obsessive-compulsive disorder. Electrodes used to apply the electrical stimulation act on the head of a patient to stimulate a designated region of the brain, thus achieving treatment of brain damage for the patient.

With the development of medical research, it is recognized that after an electrode is implanted in the brain, the contact direction and the stimulation pulse of the electrode have a significant impact on the treatment effect. As a result, there is a demand for delivering different directions and different types of electrical stimulation pulses along the same ring circumference. To improve the treatment accuracy, researchers segment the ring electrode, that is, arrange multiple segments and multiple columns of contacts on one cylindrical ring surface, aiming to target the electrical stimulation to the designated region and reduce overtreatment.

However, in the related art, when the closed-loop feedback of the pulse generator is adjusted, a contact of a segmented electrode cannot be selected in a targeted manner.

### SUMMARY

The present disclosure provides a pulse generator, a stimulator, a computer-readable storage medium and a computer program product to satisfy the requirements of practical applications.

The present disclosure provides a pulse generator. The pulse generator is configured to be implanted in a patient and includes a memory and at least one processor. The memory stores a computer program. The at least one processor is configured to, when executing the computer program, implement the following steps:

For each contact on a segmented electrode, power spectral density amplitudes of bioelectric signals corresponding to each contact delivering electrical stimulation to internal tissue of the patient and each contact not delivering electrical stimulation are respectively acquired. The power spectral density amplitudes are recorded as a first density amplitude and a second density amplitude, respectively.

One contact from multiple contacts is determined as a target contact according to the first density amplitude and the second density amplitude corresponding to each contact.

Multiple candidate contact pairs near the target contact are acquired according to a position of each contact on the segmented electrode.

When each contact does not deliver electrical stimulation, a power spectral density amplitude of a bioelectric signal corresponding to each candidate contact pair is acquired and recorded as a third density amplitude.

According to a correspondence between multiple third density amplitudes and the multiple candidate contact pairs, one candidate contact pair from the multiple candidate contact pairs is determined as the target acquisition contact pair corresponding to the target contact.

In some possible implementations, the at least one processor is configured to, when executing the computer program, acquire first density amplitudes and second density amplitudes corresponding to all the multiple contacts in the following manner:

An initial contact and an acquisition sequence of density amplitudes are acquired by utilizing the position of each contact on the segmented electrode.

The initial contact is taken as a starting point, the first density amplitude and the second density amplitude corresponding to each contact are sequentially acquired according to the acquisition sequence until the first density amplitudes and the second density amplitudes of all the multiple contacts are acquired.

Alternatively, the initial contact is taken as a starting point, the first density amplitude of each contact is sequentially acquired according to the acquisition sequence, and after the first density amplitudes of all the multiple contacts are acquired, the second density amplitude of each contact is acquired in the same sequence.

In some possible implementations, the at least one processor is configured to, when executing the computer program, acquire the first density amplitude corresponding to any contact in the following manner:

An associated contact set of the contact is acquired. The associated contact set consists of two contacts adjacent to the contact in the circumferential direction of the segmented electrode.

The electrical stimulation is delivered to internal tissue of the patient through the contact within a predetermined duration, and signal acquisition is performed on the internal tissue of the patient through the associated contact set to acquire the first density amplitude corresponding to the contact.

In some possible implementations, the at least one processor is configured to, when executing the computer program, acquire the second density amplitude corresponding to any contact in the following manner:

An associated contact set of the contact is acquired. The associated contact set consists of two contacts adjacent to the contact in the circumferential direction of the segmented electrode.

When the segmented electrode does not deliver electrical stimulation to internal tissue of the patient, signal acquisition is performed on the internal tissue of the patient through the associated contact set to acquire the second density amplitude corresponding to the contact.

In some possible implementations, the at least one processor is configured to, when executing the computer program, determine one contact from the multiple contacts as the target contact in at least one of the following manners:

A difference between the first density amplitude and the second density amplitude corresponding to each contact is acquired, and a contact corresponding to the acquired maximum difference is taken as the target contact.

For each contact and the first density amplitude and the second density amplitude corresponding to each contact, a similarity between predicted stimulation data of each contact and historical stimulation data of the segmented electrode is acquired according to the first density amplitude and the second density amplitude, the acquired similarity is taken as a first similarity, and a contact corresponding to the acquired maximum first similarity is taken as the target contact.

In some possible implementations, the at least one processor is configured to, when executing the computer program, acquire the difference between the first density amplitude and the second density amplitude corresponding to each contact, and take the contact corresponding to the acquired maximum difference as the target contact in the following manner:

A contact corresponding to a first density amplitude outside a first preset numerical range and/or a contact corresponding to a second density amplitude outside a second preset numerical range is removed.

A difference between the first density amplitude and the second density amplitude corresponding to each contact after filtering is acquired, and the contact corresponding to the maximum difference is taken as the target contact.

In some possible implementations, when the segmented electrode does not deliver electrical stimulation, for each candidate contact pair, the at least one processor is configured to, when executing the computer program, acquire the third density amplitude corresponding to each candidate contact pair in the following manner:

An eight-neighborhood search with the target contact as a center point is performed to acquire multiple contacts satisfying a preset distance and/or a preset positional relationship as candidate contacts, and two of the candidate contacts distributed on two sides of the target contact are taken as a candidate contact pair.

For each candidate contact pair, the power spectral density amplitude of the bioelectric signal corresponding to each candidate contact pair is acquired, and the power spectral density amplitude is recorded as the third density amplitude.

In some possible implementations, the at least one processor is configured to, when executing the computer program, determine the one candidate contact pair from the multiple candidate contact pairs as the target acquisition contact pair corresponding to the target contact in at least one of the following manners:

A candidate contact pair corresponding to the acquired maximum third density amplitude is taken as the target acquisition contact pair.

For each candidate contact pair and the third density amplitude corresponding to each candidate contact pair, a similarity between predicted acquisition data of each candidate contact pair and historical acquisition data of the segmented electrode is acquired according to the third density amplitude, the acquired similarity is taken as a second similarity, and a contact pair corresponding to the acquired maximum second similarity is taken as the target acquisition contact pair.

The present disclosure further provides a stimulator. The stimulator includes a segmented electrode and a pulse generator as described in any one of the above embodiments.

The segmented electrode includes an electrode lead and multiple contacts arranged in an array along the circumferential direction of the electrode lead, and each contact is configured to implement delivering electrical stimulation to internal tissue of a patient and/or acquiring a physiological signal from internal tissue of a patient.

The pulse generator is electrically connected to each contact and configured to analyze the physiological signal and generate the electrical stimulation.

In some possible implementations, the pulse generator includes a signal acquisition module, a signal processing module, a signal transmission module, and a stimulation adjustment module.

The signal acquisition module is configured to acquire the physiological signal through the multiple contacts and amplify the acquired physiological signal.

The signal processing module is configured to perform signal processing on an amplified physiological signal to acquire a power spectral density curve. The signal processing includes at least one of band-pass filtering, notch filtering and fast Fourier transform.

The signal transmission module is configured to send the amplified physiological signal to the signal processing module.

The stimulation adjustment module is configured to adjust a stimulation parameter corresponding to the electrical stimulation according to an amplitude of the physiological signal and the power spectral density curve.

The present disclosure further provides a method for determining a target contact and a target acquisition contact pair of the segmented electrode, and the method includes the following:

For each contact on a segmented electrode, power spectral density amplitudes of bioelectric signals corresponding to each contact delivering electrical stimulation to internal tissue of a patient and each contact not delivering electrical stimulation are acquired, respectively. The power spectral density amplitudes are recorded as a first density amplitude and a second density amplitude, respectively.

One contact from multiple contacts is determined as a target contact according to the first density amplitude and the second density amplitude corresponding to each contact.

Multiple candidate contact pairs near the target contact are acquired according to a position of each contact on the segmented electrode.

When each contact does not deliver electrical stimulation, a power spectral density amplitude of a bioelectric signal corresponding to each candidate contact pair is acquired and recorded as a third density amplitude.

According to a correspondence between the multiple third density amplitudes and the multiple candidate contact pairs, one candidate contact pair from the multiple candidate contact pairs is determined as the target acquisition contact pair corresponding to the target contact.

In some possible implementations, the step in which the first density amplitudes and the second density amplitudes corresponding to all contacts are acquired includes the following:

An initial contact and an acquisition sequence of density amplitudes are acquired by utilizing the position of each contact on the segmented electrode.

The initial contact is taken as a starting point, the first density amplitude and the second density amplitude corresponding to each contact are sequentially acquired according to the acquisition sequence until the first density amplitudes and the second density amplitudes of all contacts are acquired.

Alternatively, the initial contact is taken as a starting point, the first density amplitude of each contact is sequentially acquired according to the acquisition sequence, and after the first density amplitudes of all contacts are acquired, the second density amplitude of each contact is acquired in the same sequence.

In some possible implementations, the step in which the first density amplitude corresponding to any contact is acquired includes the following:

An associated contact set of the contact is acquired. The associated contact set consists of two contacts adjacent to the contact in the circumferential direction of the segmented electrode.

The electrical stimulation is delivered to internal tissue of the patient through the contact within a predetermined duration, and signal acquisition is performed on the internal tissue of the patient through the associated contact set to acquire the first density amplitude corresponding to the contact.

In some possible implementations, the step in which the second density amplitude corresponding to any contact is acquired includes the following:

An associated contact set of the contact is acquired. The associated contact set consists of two contacts adjacent to the contact in the circumferential direction of the segmented electrode.

When the segmented electrode does not deliver electrical stimulation to internal tissue of the patient, signal acquisition is performed on the internal tissue of the patient through the associated contact set to acquire the second density amplitude corresponding to the contact.

In some possible implementations, the step in which one contact from the multiple contacts is determined as the target contact includes at least one of the following:

A difference between the first density amplitude and the second density amplitude corresponding to each contact is acquired, and a contact corresponding to the acquired maximum difference is taken as the target contact.

For each contact and the first density amplitude and the second density amplitude corresponding to each contact, a similarity between predicted stimulation data of each contact and historical stimulation data of the segmented electrode is acquired according to the first density amplitude and the second density amplitude, the acquired similarity is taken as a first similarity, and a contact corresponding to the acquired maximum first similarity is taken as the target contact.

In some possible implementations, the step in which the contact corresponding to the acquired maximum difference is taken as the target contact includes the following:

A contact corresponding to a first density amplitude outside a first preset numerical range and/or a contact corresponding to a second density amplitude outside a second preset numerical range is removed.

A difference between the first density amplitude and the second density amplitude corresponding to each contact after filtering is acquired, and the contact corresponding to the maximum difference is taken as the target contact.

In some possible implementations, the step in which when the segmented electrode does not deliver electrical stimulation, for each candidate contact pair, the third density amplitude corresponding to each candidate contact pair is acquired includes the following:

An eight-neighborhood search with the target contact as a center point is performed to acquire multiple contacts satisfying a preset distance and/or a preset positional relationship as candidate contacts, and two of the candidate contacts distributed on two sides of the target contact are taken as a candidate contact pair.

For each candidate contact pair, the power spectral density amplitude of the bioelectric signal corresponding to the candidate contact pair is acquired, and the power spectral density amplitude is recorded as the third density amplitude.

In some possible implementations, the candidate contact pair from the multiple candidate contact pairs is determined as the target acquisition contact pair corresponding to the target contact in at least one of the following manners:

A candidate contact pair corresponding to the acquired maximum third density amplitude is taken as the target acquisition contact pair.

For each candidate contact pair and the third density amplitude corresponding to each candidate contact pair, a similarity between predicted acquisition data of each candidate contact pair and historical acquisition data of the segmented electrode is acquired according to the third density amplitude, the acquired similarity is taken as a second similarity, and a contact pair corresponding to the acquired maximum second similarity is taken as the target acquisition contact pair.

The present disclosure further provides a computer-readable storage medium. The computer-readable storage medium stores a computer program, when being executed by at least one processor, the computer program is configured to enable the at least one processor to implement the function of the pulse generator as described in any of the preceding implementations, or, when being executed by at least one processor, the computer program is configured to enable the at least one processor to implement the function of the stimulator as described in any of the preceding implementations, or, when being executed by at least one processor, the computer program is configured to enable the at least one processor to implement the method as described in any of the preceding implementations.

The present disclosure further provides a computer program product. The computer program product includes a computer program, when being executed by at least one processor, the computer program is configured to enable the at least one processor to implement the function of the pulse generator as described in any of the preceding implementations, or, when being executed by at least one processor, the computer program is configured to enable the at least one processor to implement the function of the stimulator as described in any of the preceding implementations, or when being executed by at least one processor, the computer program is configured to enable the at least one processor to implement the method as described in any of the preceding implementations.

### BRIEF DESCRIPTION OF DRAWINGS

FIG. 1 is a schematic flowchart of a method for determining a target contact and a target acquisition contact pair according to an embodiment of the present disclosure.
FIG. 2 is a schematic flowchart of a method for acquiring a first density amplitude and a second density amplitude according to an embodiment of the present disclosure.
Fig. 3 is another schematic flowchart of a method for acquiring a first density amplitude according to an embodiment of the present disclosure.
FIG. 4 is another schematic flowchart of a method for acquiring a second density amplitude according to an embodiment of the present disclosure.
FIG. 5 is a schematic flowchart of a method for determining a target contact according to an embodiment of the present disclosure.
FIG. 6 is a schematic flowchart of a method for acquiring a third density amplitude according to an embodiment of the present disclosure.
FIG. 7 is a schematic structural diagram of a stimulator according to an embodiment of the present disclosure.
FIG. 8 is a schematic diagram of a segmented electrode implanted in a brain of a patient according to an embodiment of the present disclosure.
FIG. 9 is a schematic structural diagram of a pulse generator according to an embodiment of the present disclosure.
FIG. 10 is a structural block diagram of an electronic device according to an embodiment of the present disclosure.
FIG. 11 is a schematic structural diagram of a computer program product according to an embodiment of the present disclosure.

### DETAILED DESCRIPTION

The technical solutions of the present disclosure are described hereinafter in conjunction with the drawings and the embodiments of the present disclosure. Under the premise of no conflict, the various embodiments or the technical features described below can be arbitrarily combined to form a new embodiment.

In the embodiments of the present disclosure, the terms "exemplarily" or "for example" is used to represent giving an example, an illustration, or a description. Any embodiment or design solution described as "exemplary" or "for example" in the embodiments of the present disclosure should not be construed as more advantageous than other embodiments or design solutions. Specifically, the use of terms such as "exemplary" or "for example" is intended to present related concepts by way of example.

Descriptions such as "first", "second" in the embodiments of the present disclosure are merely used for indicating and distinguishing between described objects, do not show a sequence, do not indicate a specific limitation on a quantity in the embodiments of the present disclosure, and do not constitute any limitation on the embodiments of the present disclosure.

The following briefly describes one application field (i.e., implantable medical devices) of the embodiments of the present disclosure.

An implantable nerve stimulation system (i.e., an implantable medical system) mainly includes a stimulator implanted in a body of a patient and a program-controlled device arranged outside the body of the patient.

Neuromodulation technology mainly involves implanting electrodes into specific structures in the body through a stereotactic surgery, and releasing electrical pulses through a stimulator implanted in the body of the patient via the electrodes to internal tissue (i.e., a target) to modulate the electrical activity and functions of the corresponding neural structure and neural network, thereby alleviating symptoms of the patient and relieving pain.

The stimulator may be any one of the following: an implantable nerve electrical stimulation device, an implantable cardiac electrical stimulation system (also known as a pacemaker), an implantable drug delivery system (IDDS), and a lead transfer apparatus. The implantable nerve electrical stimulation device is, for example, a deep brain stimulation (DBS) system, an implantable cortical nerve stimulation (CNS) system, an implantable spinal cord stimulation (SCS) system, an implantable sacral nerve stimulation (SNS) system, and an implantable vagus nerve stimulation (VNS) system.

The stimulator may include an implantable pulse generator (IPG) and segmented electrodes. An extension lead may be provided between the IPG and the segmented electrodes. The IPG is disposed in the body of the patient, and in response to a program-controlled instruction sent by the program-controlled device, the IPG provides controllable electrical stimulation energy to the internal tissue through a sealed battery and a sealed circuit. Through the implanted extension lead and the segmented electrodes, the IPG delivers one or two controllable specific electrical stimulations to a specific region of the internal tissue. The extension lead is used in combination with the IPG and acts as a medium for transmitting electrical stimulation signals to transmit the electric stimulation signals generated by the IPG to the segmented electrode. The segmented electrode delivers the electrical stimulation to the specific region of the internal tissue through multiple contacts. The stimulator is provided with one or more segmented electrodes at a single side or double sides.

It can be assumed that the segmented electrode is provided with multiple contacts, and the contacts may be uniformly arranged or non-uniformly arranged along the circumferential direction of the electrode lead. As an example, the contacts may be arranged in an array of four rows and three columns (a total of 12 contacts of the electrode) along the circumferential direction of the electrode. The contacts may include stimulation contacts and/or acquisition contacts. The contacts may be, for example, in the shape of a sheet, a ring, or a dot.

To facilitate the understanding of the segmented electrode, as an example, referring to a segmented stimulation electrode (i.e. segmented electrode) disclosed in Chinese Patent Application CN 202220209369.3, the segmented stimulation electrode includes a stimulating end and a connecting end opposite to each other, multiple electrode sheets for applying the electrical stimulation are disposed on the periphery surface of the stimulating end of the segmented stimulation electrode, multiple electrode sheets passing through the same radial plane of the segmented stimulation electrode form a group of electrode sheets, and in at least one group of electrode sheets, at least one electrode sheet has a different shape from the other electrode sheets so that multiple electrode sheets in the at least one group of electrode sheets can be distinguished from each other. As another example, referring to a segmented stimulation electrode (i.e. segmented electrode) disclosed in Chinese Patent Application CN 202210334061.6, the segmented stimulation electrode includes a inner liner and a flexible printed circuit board, the inner liner has an outer side wall for being attached with the conductive flexible circuit board; stimulation segments of the flexible printed circuit board are wrapped around the outer side wall of the inner liner, and multiple electrode sheets (i.e., contacts) of the stimulation segments are arranged on the outer side wall of the conductive flexible circuit board facing away from the inner liner; the multiple electrode sheets are divided into at least one circumferential electrode group spaced apart along the axial direction of the inner liner, and each circumferential electrode group includes multiple electrode sheets arranged along the circumferential direction.

In some optional implementations, the stimulated internal tissue may be a brain tissue of the patient, and the stimulated site may be a specific site of the brain tissue. In the case of different types of diseases among patients, the stimulated site is generally different, and the number of (single source or multi-source) contacts used, the application of one or more (single-channel or multi-channel) specific electrical stimulation signals, and stimulation parameter data are also different. The applicable disease types are not limited in the embodiments of the present disclosure, and may be the disease types applicable to DBS, SCS, pelvic stimulation, gastric stimulation, peripheral nerve stimulation, and functional electrical stimulation. DBS can be used to treat or manage the following disease types: spastic disorder (e.g., epilepsy), pain, migraine, mental disorder (e.g., major depressive disorder (MDD)), bipolar disorder, anxiety disorder, post-traumatic stress disorder, mild disorder, obsessive-compulsive disorder (OCD), behavioral disorder, mood disorder, memory disorder, physiological state disorder, movement disorder (e.g., essential tremor or Parkinson's disease), Huntington's disease, Alzheimer's disease, substance use disorder, autism, or other neurological or psychiatric diseases and impairments.

In the embodiments of the present disclosure, when the program-controlled device and the stimulator establish a program-controlled connection, the stimulation parameter of the stimulator (or the stimulation parameter of the pulse generator, different stimulation parameters correspond to different electrical stimulation signals) may be adjusted by using the program-controlled device. Alternatively, an electrophysiological activity of the patient may be sensed through the stimulator to acquire bioelectric signals, and the stimulation parameter of the stimulator can be adjusted continuously through the sensed bioelectric signals.

The stimulation parameter may include at least one of the following: electrode contact identification (e.g., 2# electrode contact and 3# electrode contact) for delivering electrical stimulation, frequency (e.g., the number of electrical stimulation pulse signals in unit time of 1 s, in Hz), pulse width (duration of each pulse, in µs), amplitude (generally expressed as voltage, i.e., the intensity of each pulse, in V), and timing (e.g., continuous timing or clustering timing, clustering timing referring to the discontinuous temporal action composed of multiple processes), stimulation mode (including one or more of current mode, voltage mode, timed stimulation mode and cyclic stimulation mode), upper and lower limits controlled by a physician (the range adjustable by the physician), and upper and lower limits controlled by the patient (the range adjustable by the patient). In an application scenario, at least one stimulation parameter of the stimulator may be adjusted in current mode or voltage mode.

The program-controlled device may be a physician program-controlled device (i.e., a program-controlled device used by the physician) or a patient program-controlled device (i.e., a program-controlled device used by the patient). The physician program-controlled device may be, for example, an intelligent terminal device equipped with a program-controlled software such as a tablet computer, a notebook computer, a desktop computer and a mobile phone. The patient program-controlled device may also be, for example, an intelligent terminal device equipped with the program-controlled software such as a tablet computer, a notebook computer, a desktop computer and a mobile phone. Furthermore, the patient program-controlled device may be other electronic devices with a program-controlled function (such as a charger and a data acquisition device with the program-controlled function).

The data interaction between the physician program-controlled device and the stimulator is not limited in the embodiments of the present disclosure. When the physician performs remote program control, the physician program-controlled device may perform data interaction with the stimulator via a server and the patient program-controlled device. When the physician performs face-to-face program control with the patient offline, the physician program-controlled device may perform data interaction with the stimulator via the patient program-controlled device. Alternatively, the physician program-controlled device may also perform data interaction with the stimulator directly.

In some possible implementations, the patient program-controlled device may include a master unit (communicating with the server) and a slave unit (communicating with the stimulator), and the master unit and the slave unit are communicatively connected. The physician program-controlled device may perform data interaction with the server over a 3rd-Generation/a 4th Generation /a 5th Generation (3G/4G/5G) mobile communication technology network. The server may perform data interaction with the master unit over the 3G/4G/5G mobile communication technology network. The master unit may perform data interaction with the slave unit via the Bluetooth protocol/wireless fidelity (WIFI) protocol/universal serial bus/(USB) protocol. The slave unit may perform data interaction with the stimulator at the working frequency band of 401 MHz to 406 MHz/2.4 GHz to 2.48 GHz. The physician program-controlled device may perform data interaction with the stimulator at the working frequency band of 401 MHz to 406 MHz/2.4 GHz to 2.48 GHz.

Power spectral density (PSD) refers to the energy distribution of a signal in the frequency domain, indicating the power or energy density of the signal at different frequencies. Spectral characteristics of a signal, including frequency components, frequency distribution and energy concentration, may be analyzed through the power spectral density. The signal may be a physiological signal, and the power spectral density may be calculated by performing Fourier transform on the acquired physiological signal. For example, the physiological signal is represented as a superposition of a series of sine and cosine components at different frequencies by converting the physiological signal in the time domain to the frequency domain through Fourier transform. In this case, the power spectral density represents the energy or power of each frequency component in the physiological signal.

In an embodiment, the actual situation of the segmented electrode is not considered by the power spectral density acquisition methods for a conventional electrode. Since the segmented electrode may be provided with contacts on multiple ring surfaces and the same ring surface may be provided with multiple contacts, as a result, the segmented electrode includes more contacts than the conventional electrode. In other words, the power spectral density acquisition method for the conventional electrode fails to consider the situation where more contacts exist on the same segmented electrode. Therefore, the power spectral density acquired by the power spectral density acquisition method for the conventional electrode still has defects in the closed-loop feedback adjustment of the segmented electrode.

On this basis, the present disclosure provides a pulse generator, a stimulator, a computer-readable storage medium and a computer program product. A target contact and a target acquisition contact pair corresponding to the target contact acquired by the pulse generator of the present disclosure can be used to determine an abnormal power spectral density. When the abnormal power spectral density is determined, the electrical stimulation pulse can be adjusted (by adjusting the stimulation parameter) so that the power spectral density is no longer abnormal, thereby achieving the closed-loop feedback adjustment for the patient. Since the adjustment data used for determining the abnormal power spectral density is acquired by using the target acquisition contact pair corresponding to the target contact, and the contact used for releasing electrical stimulation to the internal tissue of the patient is the target contact, the achieved closed-loop feedback adjustment is more personalized, the effect of electrical stimulation treatment can be improved and thus the related technologies can be improved.

### Method embodiment

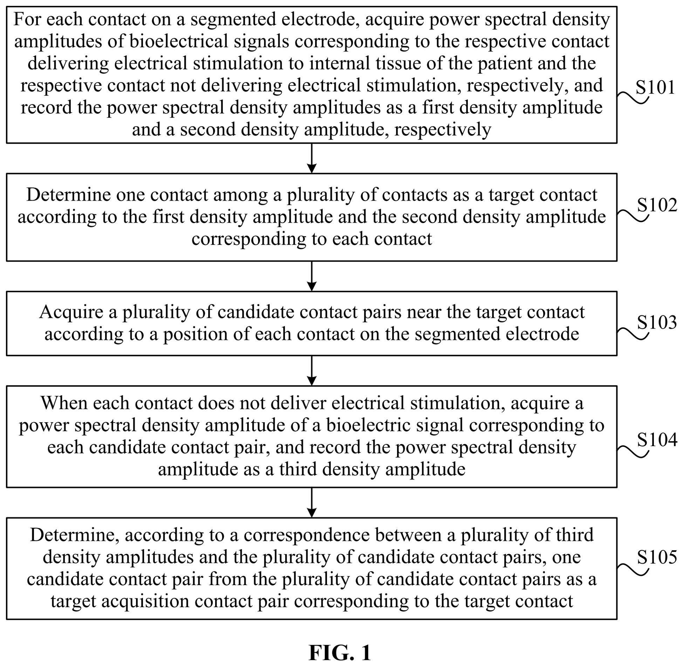

Referring to FIG. 1, FIG. 1 is a schematic flowchart of a method for determining a target contact and a target acquisition contact pair according to an embodiment of the present disclosure.

The pulse generator involved in the method embodiment is configured to be implanted in a patient. The method is used to determine the target contact and the target acquisition contact pair of a segmented electrode. The method includes the following steps.

In step S101, for each contact on a segmented electrode, power spectral density amplitudes of bioelectric signals corresponding to the respective contact delivering electrical stimulation to internal tissue of the patient and the respective contact not delivering electrical stimulation are respectively acquired. The power spectral density amplitudes are recorded as a first density amplitude and a second density amplitude, respectively.

In step S102, one contact from multiple contacts is determined as a target contact according to the first density amplitude and the second density amplitude corresponding to each contact.

In step S103, multiple candidate contact pairs near the target contact are acquired according to a position of each contact on the segmented electrode.

In step S104, when each contact does not deliver electrical stimulation, a power spectral density amplitude of a bioelectric signal corresponding to each candidate contact pair is acquired and recorded as a third density amplitude.

In step S105, according to a correspondence between the multiple third density amplitudes and the multiple candidate contact pairs, one candidate contact pair from the multiple candidate contact pairs is determined as the target acquisition contact pair corresponding to the target contact.

The present disclosure is not limited to the type of the patient for which the pulse generator is used, for example, a patient with Parkinson's disease, or a patient with obsessive-compulsive disorder. The following description takes Parkinson's disease as an example to facilitate understanding. Parkinson's disease is a neurodegenerative disease whose main symptoms are movement disorders, such as tremor, bradykinesia and myotonia. The bioelectric signal to be acquired by the pulse generator is, for example, a local field potential (LFP) signal. Parkinson's disease is typically characterized by the power spectral density of the LFP signal at *β* frequency band (13 Hz to 35 Hz) being higher or lower than a preset range, i.e., abnormal power spectral density. That is to say, determination of whether the power spectral density is abnormal may be used as an important basis for closed-loop feedback of the patient with Parkinson's disease and may be used to adjust the electrical stimulation parameter for the patient to alleviate the symptoms of the patient with Parkinson's disease. It can be understood that for other diseases (such as obsessive-compulsive disorder), the power spectral density of the frequency band corresponding to the disease may also be acquired by acquiring the bioelectric signal from the patient, and then the power spectral density is used for closed-loop feedback of the patient.

Therefore, in the technical solution of the present embodiment, for each contact on the segmented electrode, the signal energy distribution when the electrical stimulation is delivered and not delivered is measured and recorded. The first density amplitude and the second density amplitude corresponding to each contact are compared to determine one of the contacts as the target contact. The target contact determined after measurement and comparison may be used as a contact for delivering the electrical stimulation on the segmented electrode. According to the position of the target contact, multiple candidate contact pairs on the segmented electrode near the target contact are acquired. When all contacts do not deliver electrical stimulation, the third density amplitude corresponding to each candidate contact pair is acquired, and each third density amplitude is compared to determine the target acquisition contact pair. The target acquisition contact pair may be used as contacts determined for acquiring the bioelectric signal for the target contact point.

Through the above steps, an appropriate target contact and target acquisition contact pair can be automatically determined to enable the pulse generator to deliver electrical stimulation more accurately while avoiding possible errors or inaccuracies caused by manual determination. Exemplarily, at least the following effects are obtained:

In an aspect, based on the comparison between the first density amplitude and the second density amplitude, the target contact is selected from multiple contacts, and the target acquisition contact pair corresponding to the target contact is determined, so the targeted stimulation therapy with closed-loop feedback adjustment can be performed on the patient only through the acquired target contact and target acquisition contact pair, thereby improving the pertinence and effect of the treatment. Furthermore, unnecessary electrical stimulation delivered by contacts and bioelectric signal acquisition are reduced, and the damage and impact of the pulse generator and the segmented electrode on the internal tissue of the patient can be reduced.

In another aspect, for the third density amplitude, a local field potential signal of each candidate contact pair is acquired only when the segmented electrode does not deliver electrical stimulation, so that the interference of electrical stimulation on the signal acquisition process can be avoided. This is because an additional electrical activity is introduced when electrical stimulation is applied, the characteristics of bioelectric signals may be interfered with and changed by the additional electrical activity, thus affecting the acquisition of the third density amplitude. Therefore, a clearer and more accurate local field potential signal can be acquired since the signal acquisition is performed when the segmented electrode does not deliver the electrical stimulation, which can better reflect the actual activity of the target contact in the internal tissue of the patient.

To sum up, the pulse generator of the embodiments of the present disclosure can accurately locate the target contact and determine the target contact from multiple contacts by analyzing the power spectral density amplitude of the bioelectric signal corresponding to each contact, thus improving the pertinence and effect of the treatment. Furthermore, power spectral density amplitudes of bioelectric signals of candidate contact pairs are acquired and compared with the third density amplitude so that the most suitable candidate contact pair can be determined, and the accuracy and effect of treatment can be improved. Therefore, the technical solution can greatly improve the accuracy and efficiency of using the pulse generator to deliver the electrical stimulation to the segmented electrode, and a better therapeutic effect can be achieved.

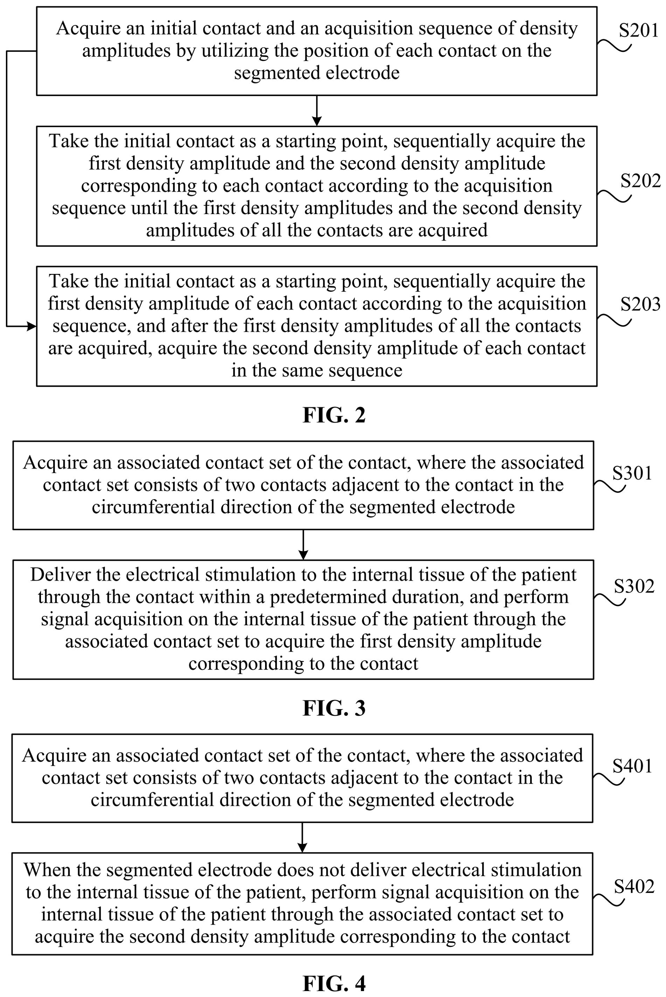

Referring to FIG. 2, FIG. 2 is a schematic flowchart of acquiring a first density amplitude and a second density amplitude according to an embodiment of the present disclosure.

In some embodiments, acquiring the first density amplitude and the second density amplitude corresponding to all the multiple contacts may include the following steps.

In step S201, an initial contact and an acquisition sequence of density amplitudes are acquired by utilizing the position of each contact on the segmented electrode.

In step S202, the initial contact is taken as a starting point, and the first density amplitude and the second density amplitude corresponding to each contact are sequentially acquired according to the acquisition sequence until the first density amplitudes and the second density amplitudes of all the contacts are acquired.

Alternatively, in step S203, the initial contact is taken as a starting point, the first density amplitude of each contact is sequentially acquired according to the acquisition sequence, and after the first density amplitudes of all contacts are acquired, the second density amplitude of each contact is acquired in the same sequence.

Therefore, according to the position of each contact on the segmented electrode, the acquisition sequence for the contact is determined, i.e., the initial contact and the acquisition sequence of the density amplitudes are determined. In one aspect, the consistency and accuracy of data can be ensured by acquiring the density amplitude of each contact in a specific sequence. In another aspect, the first density amplitude and the second density amplitude corresponding to each contact are acquired so that more comprehensive signal energy distribution information can be acquired, facilitating the analysis and evaluation of contact applicability. In another aspect, in the application, the first density amplitude and the second density amplitude can be acquired one by one; alternatively, the first density amplitude can be acquired first, followed by the second density amplitude; that is, the density amplitude acquisition method is flexible, so that different treatment strategies and analysis requirements can be satisfied. In another aspect, the density amplitudes of multiple contacts on the segmented electrode are acquired so that the target contact can be located more accurately. In another aspect, on the basis of accurately locating the target contact, the target acquisition contact pair acquired according to the target contact can provide a more objective reflection of the stimulation effect.

To sum up, the pulse generator according to the present embodiment acquires the density amplitude of each contact according to the acquisition sequence of density amplitudes after the initial contact is acquired, provides different acquisition methods, offers flexibility and comprehensiveness, and facilitates more accurate evaluation and determination of the target contact, thus improving the treatment effect and adaptability.

The present embodiment does not limit the acquisition sequence of density amplitudes. The acquisition sequence may be, for example, a sequence preset by the physician or an acquisition sequence determined according to the distance relationship between other contacts and the initial contact.

The initial contact may be any contact on the outermost ring surface along the axial direction of the electrode or any contact on another ring surface along the axial direction of the electrode. When the initial contact is any contact on the outermost ring surface along the axial direction of the electrode, the acquisition sequence may start with each contact on the ring surface where the initial contact is located, then proceed to each contact on an adjacent ring surface until the first density amplitudes and the second density amplitudes of all contacts are acquired. When the initial contact is not the contact on the outermost ring surface along the axial direction of the electrode, the acquisition sequence may be each contact on the ring surface where the initial contact is located, then proceed to each contact on the ring surface adjacent in one direction, and then each contact on the ring surface adjacent in the other direction until the first density amplitudes and the second density amplitudes of all contacts are acquired.

Starting from the initial contact, the first density amplitude and the second density amplitude corresponding to each contact are sequentially acquired according to the acquisition sequence of the density amplitudes until the first density amplitudes and the second density amplitudes corresponding to all contacts are acquired. In this manner, the signal energy distribution information of the contacts may be acquired one by one. Alternatively, starting from the initial contact point, the first density amplitude of each contact is sequentially acquired until the first density amplitudes of all contacts are acquired. Then, the second density amplitude of each contact is acquired in the same sequence. In this manner, the signal energy distribution information of the contacts may be acquired step by step.

Referring to FIG. 3, FIG. 3 is another schematic flowchart of acquiring the first density amplitude according to an embodiment of the present disclosure.

In some embodiments, acquiring the first density amplitude corresponding to any contact may include the following steps.

In step S301, an associated contact set of the contact is acquired. The associated contact set consists of two contacts adjacent to the contact in the circumferential direction of the segmented electrode.

In step S302, the electrical stimulation is delivered to the internal tissue of the patient through the contact within a predetermined duration, and signal acquisition is performed on the internal tissue of the patient through the associated contact set to acquire the first density amplitude corresponding to the contact.

Therefore, two adjacent contacts of each contact in the circumferential direction of the segmented electrode are determined to form an associated contact set. These associated contact sets represent contacts adjacent to the target contact. The electrical stimulation is delivered to the internal tissue of the patient through the target contact within the predetermined duration. At the same time, the signal acquisition is performed on the internal tissue of the patient through the associated contact set. Through the acquired signal, the first density amplitude corresponding to the target contact is acquired by calculation and reflects the signal energy distribution at the position of the contact.

In summary, the first density amplitude of the target contact is acquired by utilizing the associated contact set and the local field potential signal acquisition. The present embodiment provides an effective method to evaluate the signal energy distribution of the target contact, offering a useful reference for adjustment of the pulse generator and formulation of the treatment strategy.

The present embodiment does not limit the predetermined duration, which, for example, may be 30 seconds, 1 minute, or 15 minutes.

Referring to FIG. 4, FIG. 4 is another schematic flowchart of acquiring the second density amplitude according to an embodiment of the present disclosure.

In some embodiments, acquiring the second density amplitude corresponding to any contact may include the following steps.

In step S401, an associated contact set of the contact is acquired. The associated contact set consists of two contacts adjacent to the contact in the circumferential direction of the segmented electrode.

In step S402, when the segmented electrode does not deliver electrical stimulation to the internal tissue of the patient, signal acquisition is performed on the internal tissue of the patient through the associated contact set to acquire the second density amplitude corresponding to the contact.

Therefore, two adjacent contacts of each contact in the circumferential direction of the segmented electrode are determined to form an associated contact set. These associated contact sets represent contacts adjacent to the target contact. The signal acquisition is performed on the internal tissue of the patient through the associated contact set only when the segmented electrode does not deliver electrical stimulation to the internal tissue of the patient. Through the acquired signal, the second density amplitude corresponding to the target contact is acquired by calculation and reflects the signal energy distribution at the position of the contact.

In summary, the second density amplitude of the target contact is acquired by utilizing the associated contact set and the local field potential signal acquisition, and the acquired second density amplitude can be used more effectively to evaluate the signal energy distribution of the target contact, providing a useful reference for the adjustment of the pulse generator.

In some embodiments, determining one contact from the multiple contacts as the target contact may include at least one of the following steps.

A difference between the first density amplitude and the second density amplitude corresponding to each contact is acquired, and a contact corresponding to the acquired maximum difference is taken as the target contact.

For each contact and the first density amplitude and the second density amplitude corresponding to each contact, a similarity between predicted stimulation data of each contact and historical stimulation data of the segmented electrode is acquired according to the first density amplitude and the second density amplitude, the acquired similarity is taken as a first similarity, and a contact corresponding to the acquired maximum first similarity is taken as the target contact.

Therefore, the target contact may be determined by comparing the difference between the first density amplitude and the second density amplitude. The first density amplitude and the second density amplitude corresponding to the contact are acquired, and the difference between the first density amplitude and the second density amplitude corresponding to the contact is calculated. The contact with the acquired maximum difference is determined by comparison and is taken as the target contact. It can be understood that the contact with the maximum difference has a stronger bioelectric signal so that the data acquisition can be more accurate. Alternatively, the target contact may also be determined by analyzing the similarity between the predicted stimulation data and the historical stimulation data. For each contact and the first density amplitude and the second density amplitude corresponding to each contact, the similarity between the historical stimulation data of the segmented electrode and the predicted stimulation data corresponding to the contact may be calculated according to the first density amplitude and the second density amplitude, and the acquired similarity is taken as the first similarity. The contact with the acquired maximum first similarity is taken as the target contact. The present embodiment does not limit the acquisition method of the similarity, which is, for example, cosine similarity or Euclidean distance. The optimal target contact is determined by comparing with the historical data, thereby acquiring more accurate bioelectric signal data.

In some embodiments, the predicted stimulation data is acquired in the following manner: a density amplitude group (consisting of the first density amplitude and the second density amplitude) is input into a prediction model to acquire predicted stimulation data of the density amplitude group.

A training process of the prediction model includes the following:

A first training set is acquired. The first training set includes multiple training data, and each training data includes a sample density amplitude group and label data for the predicted stimulation data of the sample density amplitude group.

For each training data in the first training set, the following processing is performed:

The sample density amplitude group in the training data is input into a preset first deep learning model to acquire predicted data for the predicted stimulation data of the sample density amplitude group.

A model parameter of the first deep learning model is updated based on the prediction data and the label data.

It is detected whether a preset training termination condition is satisfied. If yes, the trained first deep learning model is taken as the prediction model. If not, the first deep learning model is continuously trained by using the next training data.

Therefore, an appropriate number of neural computation nodes and a multi-layer computational hierarchy are designed and established, and an appropriate input layer and an appropriate output layer are selected so that the preset first deep learning model can be acquired. Through learning and optimization of the preset first deep learning model, a functional relationship from input to output can be established. Although the functional relationship between input and output cannot be determined with 100% probability, the functional relationship can be as close to the correlation in reality as possible. In this manner, the prediction model acquired through the present training can acquire predicted stimulation data based on the density amplitude group, and the calculation result has high accuracy and high reliability.

The present embodiment does not limit the method of acquiring the label data for the predicted stimulation data. For example, manual labeling, automatic labeling, or semi-automatic labeling may be adopted.

The present embodiment does not limit the training process of the prediction model. For example, the above supervised learning training mode, semi-supervised learning training mode, or unsupervised learning training mode may be adopted.

The present embodiment does not limit the preset training termination condition, which may be that, for example, the number of training epochs reaches a preset number (e.g., 1, 3, 10, 100, 1,000, 10,000), or all the training data in the first training set are trained for one or more epochs, or the total loss value acquired in the current training is not greater than a preset loss value.

In some embodiments, the first similarity is acquired in the following manner: predicted stimulation data and historical stimulation data are input into a similarity model to acquire the first similarity between the predicted stimulation data and the historical stimulation data.

A training process of the similarity model includes the following:

A second training set is acquired. The second training set includes multiple training data, and each training data includes first sample stimulation data, second sample stimulation data and label data of a similarity between the first sample stimulation data and the second sample stimulation data.

For each training data in the second training set, the following processing is performed:

The first sample stimulation data and the second sample stimulation data in the training data are input into a preset second deep learning model to acquire predicted data of the similarity between the first sample stimulation data and the second sample stimulation data.

A model parameter of the second deep learning model is updated based on the predicted data and the label data of the similarity between the first sample stimulation data and the second sample stimulation data.

It is detected whether a preset training termination condition is satisfied. If so, the trained second deep learning model is taken as the similarity model. If not, the second deep learning model is continuously trained by using the next training data.

Therefore, the similarity model can be trained with a large amount of training data and predict the corresponding output data for different input data, which has a wide application range and a high intelligence level. An appropriate number of neural computation nodes and a multi-layer computational hierarchy are designed and established, and an appropriate input layer and an appropriate output layer are selected so that the preset second deep learning model can be acquired. Through learning and optimization of the preset second deep learning model, a functional relationship from input to output can be established. Although the functional relationship between input and output cannot be determined with 100% probability, the functional relationship can be as close to the correlation in reality as possible. In this manner, the similarity model acquired through the present training can acquire the similarity between the predicted stimulation data of each contact and the historical stimulation data of the segmented electrode based on the first density amplitude and the second density amplitude, and the calculation result has high accuracy and high reliability.

In some alternative implementations, the present disclosure can use the above training process to train the similarity model. In other alternative implementations, the present disclosure can use a pre-trained similarity model.

In some optional implementations, for example, data mining may be performed on the historical data to acquire training data. The first sample stimulation data and the second sample stimulation data may also be automatically generated by using a generative network of a generative defensive network (GAN) model.

The GAN model, a generative defensive network, consists of a generative network and a discriminative network. The generative network randomly samples data from latent space as input, and an output result of the generative network is required to mimic a real sample in the second training set as closely as possible. The discriminator network takes a real sample or the output of the generative network as input, aiming to distinguish the output of the generative network from the real sample as much as possible. On the other hand, the generative network needs to deceive the discriminative network as much as possible. The two networks defend each other and constantly adjust the corresponding parameter, and the ultimate goal is to make it impossible for the discriminative network to determine whether the output of the generative network is genuine. The first sample stimulation data and the second sample stimulation data generated by using the GAN model can be used in the training process of the similarity model, effectively reducing the data amount of raw data acquisition and significantly lowering the cost of data acquisition and labeling.

The present embodiment does not limit the method of acquiring the label data. For example, manual labeling, automatic labeling, or semi-automatic labeling may be adopted.

The present embodiment does not limit the training process of the similarity model. For example, the above supervised learning training mode, semi-supervised learning training mode, or unsupervised learning training mode may be adopted.

The present embodiment does not limit the preset training termination condition, which may be that, for example, the number of training epochs reaches a preset number (e.g., 1, 3, 10, 100, 1,000, 10,000), or all the training data in the second training set are trained for one or more epochs, or the total loss value acquired in the current training is not greater than a preset loss value.

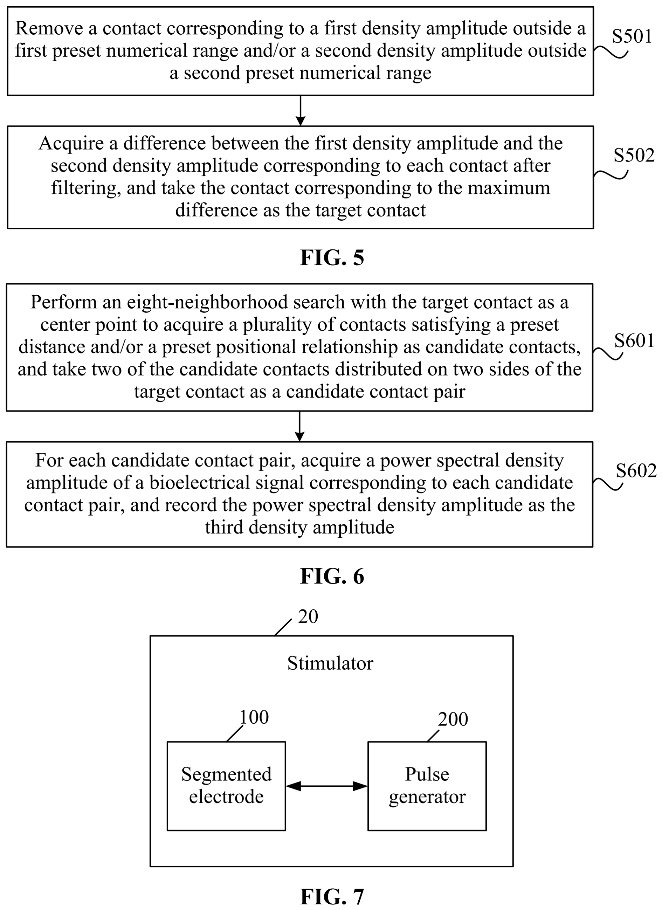

Referring to FIG. 5, FIG. 5 is a schematic flowchart of determining a target contact according to an embodiment of the present disclosure.

In some embodiments, taking the contact corresponding to the acquired maximum difference as the target contact may include the following steps.

In step S501, a contact corresponding to a first density amplitude outside a first preset numerical range and/or a contact corresponding to a second density amplitude outside a second preset numerical range is removed.

In step S502, a difference between the first density amplitude and the second density amplitude corresponding to each contact after filtering is acquired, and the contact corresponding to the maximum difference is taken as the target contact.

Therefore, the bioelectric signals of multiple contacts are acquired and processed to acquire the difference between the first density amplitude and the second density amplitude corresponding to each contact, the data outside the preset numerical range is filtered out, and the contact corresponding to the maximum difference is determined as the target contact.

In an aspect, through acquisition and processing of physiological signals, an accurate density amplitude difference can be acquired to determine the target contact, reducing the risk of error and missed diagnosis, and achieving high accuracy. In another aspect, different contacts induce different bioelectric signal responses in the internal tissue of the patient. Compared with determining the target contact by considering the differences between the first density amplitudes or the second density amplitudes corresponding to different contacts, the present embodiment can improve the comparison effect of signal and noise by calculating the difference of power spectral density amplitudes before and after stimulation. The target contact may exhibit a large amplitude variation before and after stimulation, while other non-target contacts may have relatively small variations, thus making the target contact easier to identify. In another aspect, the difference between the power spectral density amplitudes of the same contact before and after stimulation is calculated so that the difference between the target contact and other contacts can be amplified, facilitating the accurate determination of the target contact while reducing the influence of other contacts.

The present embodiment does not limit the first preset numerical range and the second preset numerical range, each of which may be, for example, any one of [1, 3], [0.8, 2], or [1.2, 4].

Referring to FIG. 6, FIG. 6 is a schematic flowchart of acquiring a third density amplitude according to an embodiment of the present disclosure.

In some embodiments, when the segmented electrode does not deliver electrical stimulation, for each candidate contact pair, acquiring a third density amplitude corresponding to each candidate contact pair may include the following steps.

In step S601, an eight-neighborhood search with the target contact as a center point is performed to acquire multiple contacts satisfying a preset distance and/or a preset positional relationship as candidate contacts, and two of the candidate contacts distributed on two sides of the target contact are taken as a candidate contact pair.

In step S602, for each candidate contact pair, the power spectral density amplitude of the bioelectrical signal corresponding to the candidate contact pair is acquired and recorded as the third density amplitude.

The eight-neighborhood search may be understood as searching for the nearest contact in eight directions, i.e., up, down, left, right, upper left, lower left, upper right, and lower right directions around the target contact by using the target contact as the center point.

In the present embodiment, the eight-neighborhood search with the target contact as the center point is performed to acquire the multiple contacts satisfying the preset distance and/or the preset positional relationship as the candidate contacts, and two of the candidate contacts distributed on the two sides of the target contact are taken as the candidate contact pair. For each candidate contact pair, the power spectrum density amplitude of the bioelectrical signal corresponding to the candidate contact pair is acquired and recorded as the third density amplitude. In one aspect, the multiple candidate contacts satisfying the preset conditions can be acquired by performing the eight-neighborhood search with the target contact as the center point. In this manner, the determination range of the candidate contacts is increased, and the determination accuracy of the candidate contacts is improved. In another aspect, the contacts on the two sides of the target contact are specifically considered when the candidate contact pair is determined, which facilitates the determination of the positional relationship of the candidate contact pairs and improves the accuracy of the contact selection. In another aspect, the power spectral density amplitude of the bioelectric signal corresponding to the candidate contact pair, i.e., the third density amplitude, is acquired so that the candidate contact pair can be compared and evaluated, which facilitates the determination of the most suitable contact pair as the target acquisition contact pair.

The present disclosure does not limit the preset distance and the preset position. The preset distance may be, for example, 1 mm, 1.1 mm, or 1.4 mm. The contact satisfying the preset position may be, for example, at least one contact in the upper left direction, the lower left direction, the upper right direction, or the lower right direction, with the target contact as the center point.

In some embodiments, one candidate contact pair is determined from the candidate contact pairs as the target acquisition contact pair corresponding to the target contact in at least one of the following manners:

A candidate contact pair corresponding to the acquired maximum third density amplitude is taken as the target acquisition contact pair.

For each candidate contact pair and the third density amplitude corresponding to each candidate contact pair, a similarity between predicted acquisition data of each candidate contact pair and historical acquisition data of the segmented electrode is acquired according to the third density amplitude, the acquired similarity is taken as a second similarity, and a contact pair corresponding to the acquired maximum second similarity is taken as the target acquisition contact pair.

Therefore, the candidate contact pair corresponding to the acquired maximum third density amplitude may be taken as the target acquisition contact pair. Since the third density amplitude represents the power spectrum density amplitude of the bioelectric signal corresponding to the respective candidate contact pair, the target acquisition contact pair may be determined by comparing the magnitudes of these density amplitudes. Furthermore, according to the similarity between the predicted acquisition data and the historical acquisition data, a candidate contact pair corresponding to the maximum similarity may be determined as the target acquisition contact pair.

In one aspect, when the candidate contact pair corresponding to the maximum third density amplitude is determined as the target acquisition contact pair, it facilitates capturing the most significant signal variation of the target contact through the target acquisition contact pair.

In another aspect, the adaptability and consistency of each candidate contact pair can be evaluated by calculating the similarity between the predicted acquisition data and the historical acquisition data. When the contact pair corresponding to the maximum second similarity is determined as the target acquisition contact pair, the data acquired through the target acquisition contact pair can be more stable and reliable.

In the present embodiment, the second similarity may be acquired in a manner similar to the manner of acquiring the first similarity, which will not be repeated here.

In some embodiments, before step S 101 is executed, the method may further include the following steps.

User data of the patient is acquired, and the user data includes disease type information of the patient.

Frequency band parameters corresponding to any of the first density amplitude, the second density amplitude and the third density amplitude are determined according to the user data. The frequency band parameters are used to indicate the frequency band acquired during signal acquisition.

In Parkinson's disease, the electroencephalographic activity of the patient typically exhibits abnormal power spectral density within a specific frequency range. In an LFP signal, the patient with Parkinson's disease often shows an abnormally elevated power spectral density at *β* band (13 Hz to 35 Hz).

The abnormally elevated power spectral density is associated with the abnormal neural activity of the patient with Parkinson's disease. It can be assumed that Parkinson's disease is related to the abnormal firing activity of basal neurons in the brain of the patient, particularly an abnormally elevated firing activity at *β* band. The abnormal firing activity may lead to an increase in the power spectral density at *β* band, which in turn is related to the symptoms of Parkinson's disease through the density amplitude with the frequency band parameter of *β*.

Therefore, the power spectral density amplitude, particularly the power spectral density amplitude at *β* band, of the LFP signal is analyzed, which facilitates the evaluation of the severity of the disease and provides guidance for the diagnosis and treatment of the disease.

Therefore, in one aspect, a signal band of interest is filtered and determined by determining the specific frequency band for anomaly determination, thus reducing the amount of data to be processed and analyzed. Compared with analyzing the entire spectrum range, only the specific frequency band is focused on so that the calculation efficiency can be improved and the computational cost is reduced. In another aspect, some diseases or symptoms may have specific spectral characteristics. A signal characteristic related to a pathological state can be focused on by performing anomaly determination for the specific frequency band, improving the sensitivity and specificity of anomaly detection. In another aspect, the pathological-related frequency band is focused on so that the accuracy of anomaly determination can be improved. This is because the frequency band related to the specific disease is focused on, enabling the interference of irrelevant noise to be reduced, and enhancing the detection ability of the abnormal signal. In another aspect, spectrum analysis and power spectrum density calculation are computationally intensive tasks. The required computational load and complexity can be reduced by limiting the frequency range for analysis, thus lowering computational complexity and improving the execution efficiency.

To sum up, the frequency band for anomaly determination is determined according to the symptoms so that the data processing and analysis process can be optimized, the computational load can be reduced, and the accuracy and efficiency of anomaly detection can be improved.

In an application scenario, the embodiments of the present disclosure further provide a method for determining a target contact and a target acquisition contact pair of a segmented electrode, and the method includes the following steps:

For each contact on a segmented electrode, an initial contact and an acquisition sequence of density amplitudes are acquired by utilizing the position of each contact on the segmented electrode.

The initial contact is taken as a starting point, and the first density amplitude and the second density amplitude corresponding to each contact are sequentially acquired according to the acquisition sequence until the first density amplitudes and the second density amplitudes of all contacts are acquired.

Alternatively, the initial contact is taken as a starting point, the first density amplitude of each contact is sequentially acquired according to the acquisition sequence, and after the first density amplitudes of all contacts are acquired, the second density amplitude of each contact is acquired in the same sequence.

One contact from multiple contacts is determined as a target contact according to the first density amplitude and the second density amplitude corresponding to each contact.

Multiple candidate contact pairs near the target contact are acquired according to the position of each contact on the segmented electrode.

When each contact does not deliver electrical stimulation, a power spectral density amplitude of a bioelectric signal corresponding to each candidate contact pair is acquired and recorded as a third density amplitude.

According to a correspondence between multiple third density amplitudes and the multiple candidate contact pairs, one candidate contact pair from the multiple candidate contact pairs is determined as the target acquisition contact pair corresponding to the target contact.

The process of acquiring the first density amplitude corresponding to any contact includes the following:

An associated contact set of the contact is acquired, and the associated contact set consists of two contacts adjacent to the contact in the circumferential direction of the segmented electrode.

The electrical stimulation is delivered to the internal tissue of the patient through the contact within a predetermined duration, and signal acquisition is performed on the internal tissue of the patient through the associated contact set to acquire the first density amplitude corresponding to the contact.

The process of acquiring the second density amplitude corresponding to any contact includes the following:

An associated contact set of the contact is acquired. The associated contact set consists of two contacts adjacent to the contact in a circumferential direction of the segmented electrode.

When the segmented electrode does not deliver electrical stimulation to internal tissue of the patient, signal acquisition is performed on the internal tissue of the patient through the associated contact set to acquire the second density amplitude corresponding to the contact.

The process of determining one contact from the multiple contacts as a target contact includes the following:

A contact corresponding to a first density amplitude outside a first preset numerical range and/or a contact corresponding to a second density amplitude outside a second preset numerical range is removed.

A difference between the first density amplitude and the second density amplitude corresponding to each contact after filtering is sequentially acquired, and the contact corresponding to the maximum difference is taken as the target contact.

The process of determining one contact from the multiple contacts as a target contact may further include the following:

For each contact and the first density amplitude and the second density amplitude corresponding to each contact, a similarity between predicted stimulation data of each contact and historical stimulation data of the segmented electrode is sequentially acquired according to the first density amplitude and the second density amplitude, the acquired similarity is taken as a first similarity, and a contact corresponding to the acquired maximum first similarity is taken as the target contact.

When the segmented electrode does not deliver electrical stimulation, for each candidate contact pair, the process of acquiring the third density amplitude corresponding to the candidate contact pair includes the following:

An eight-neighborhood search with the target contact as a center point is performed to acquire multiple contacts satisfying a preset distance and/or a preset positional relationship as candidate contacts, and two of the candidate contacts distributed on two sides of the target contact are taken as a candidate contact pair.

For each candidate contact pair, the power spectral density amplitude of the bioelectrical signal corresponding to the candidate contact pair is acquired and recorded as the third density amplitude.

The process of determining one candidate contact pair from the multiple candidate contact pairs as the target acquisition contact pair corresponding to the target contact includes at least one of the following:

A candidate contact pair corresponding to the acquired maximum third density amplitude is taken as the target acquisition contact pair.

For each candidate contact pair and the third density amplitude corresponding to each candidate contact pair, a similarity between predicted acquisition data of each candidate contact pair and historical acquisition data of the segmented electrode is acquired according to the third density amplitude, the acquired similarity is taken as a second similarity, and a contact pair corresponding to the acquired maximum second similarity is taken as the target acquisition contact pair.

In an application, three segmented electrodes are implanted in the brain of patient little A with Parkinson's disease, which are designated as 1# electrode, 2# electrode, and 3# electrode, respectively.

1# electrode, 2# electrode and 3# electrode are all segmented electrodes, and 1# electrode is a segmented electrode in 3-3-3-3 configuration, where 3-3-3-3 means that 1# electrode includes four contact rings, each contact ring includes three contacts, and 1# electrode includes twelve contacts in total, which are respectively designated as 1a, 1b, 1c, 2a, 2b, 2c, 3a, 3b, 3c, 4a, 4b, and 4c. It can be understood that 1a, 1b and 1c are contacts in the same contact ring, 2a, 2b and 2c are contacts in the same contact ring, 3a, 3b and 3c are contacts in the same contact ring, and 4a, 4b and 4c are contacts in the same contact ring. 1a, 2a, 3a and 4a are contacts in the same column along the axial direction of the segmented electrode, 1b, 2b, 3b and 4b are contacts in the same column along the axial direction of the segmented electrode, and 1c, 2c, 3c and 4c are contacts in the same column along the axial direction of the segmented electrode. Each contact may be configured for stimulation and acquisition, respectively. Personal information of patient little A is acquired, and the personal information includes the disease type of patient little A being Parkinson's disease. According to the disease type, the current stimulation parameter corresponding to 1# electrode is acquired as follows: amplitude of 3 V, pulse width of 120 µs, and frequency of 130 Hz.

Cyclic stimulation and data acquisition are performed on the contacts of 1# electrode, including 12 situations, including the following situations: stimulation through 1a with data acquisition through 1b and 1c, stimulation through 1b with data acquisition through 1a and 1c, ... stimulation through 4c with data acquisition through 4a and 4b. In each situation, the state of data acquisition without stimulation and the state of data acquisition with stimulation are maintained for 1 minute, respectively. Average values of the power spectral density amplitudes of the LFP signal at *β* band in the state of data acquisition without stimulation are recorded as A1, A2, A3, ... A12. Average values of the power spectral density amplitudes of the LFP signal at *β* band in the state of data acquisition with stimulation are recorded as B1, B2, B3, ... B12. The situation corresponding to the maximum amplitude difference (Ai - Bi, i = 1 to 12) in both states is determined as the optimal stimulation contact (i.e., the target contact mentioned above).

Assuming that 2b is the optimal stimulation contact, the symmetrical adjacent contacts 2a and 2c, 1a and 3c, 1b and 3b, 1c and 3a around the optimal stimulation contact 2b are determined to perform data acquisition without stimulation, respectively, the average values of the power spectral density amplitudes of the LFP signal at β band are acquired and recorded as C1, C2, C3 and C4, respectively, and the symmetrical contacts corresponding to the maximum average value are determined as the optimal LFP acquisition contacts. Similarly, the same operation may be performed on 2# electrode and 3# electrode to acquire the target contact and the target acquisition contact pair corresponding to the respective electrode.

### Pulse generator embodiment

The embodiments of the present disclosure further provide a pulse generator. The implementations of the pulse generator are consistent with the implementations described in method embodiment, the same technical effect as the method is achieved, and some details will not be repeated here.

The pulse generator is configured to be implanted in a patient. The pulse generator includes a memory and at least one processor. The memory stores a computer program, and the at least one processor is configured to, when executing the computer program, perform the following steps.

In step S101, for each contact on a segmented electrode, the power spectral density amplitudes of the bioelectric signals corresponding to the contact delivering electrical stimulation to internal tissue of the patient and the contact not delivering electrical stimulation are acquired, respectively. The power spectral density amplitudes are recorded as a first density amplitude and a second density amplitude, respectively.

In step S102, one contact from multiple contacts is determined as a target contact according to the first density amplitude and the second density amplitude corresponding to each contact.

In step S103, multiple candidate contact pairs near the target contact are acquired according to a position of each contact on the segmented electrode.

In step S104, when each contact does not deliver electrical stimulation, a power spectral density amplitude of a bioelectric signal corresponding to each candidate contact pair is acquired and recorded as a third density amplitude.

In step S105, according to a correspondence between multiple third density amplitudes and the multiple candidate contact pairs, one candidate contact pair from the multiple candidate contact pairs is determined as the target acquisition contact pair corresponding to the target contact.

In some embodiments, the at least one processor may be configured to, when executing the computer program, acquire first density amplitudes and second density amplitudes corresponding to all contacts in the following manner:

In step S201, an initial contact and an acquisition sequence of density amplitudes are acquired by utilizing the position of each contact on the segmented electrode.

In step S202, the initial contact is taken as a starting point, the first density amplitude and the second density amplitude corresponding to each contact are sequentially acquired according to the acquisition sequence until the first density amplitudes and the second density amplitudes of all the multiple contacts are acquired; alternatively/optionally,

in step S203, the initial contact is taken as a starting point, the first density amplitude of each contact is sequentially acquired according to the acquisition sequence, and after the first density amplitudes of all contacts are acquired, the second density amplitude of each contact is acquired in the same sequence.

In some embodiments, the at least one processor may be configured to, when executing the computer program, acquire the first density amplitude corresponding to any contact in the following manner:

In step S301, an associated contact set of the contact is acquired. The associated contact set consists of two contacts adjacent to the contact in a circumferential direction of the segmented electrode.

In step S302, the electrical stimulation is delivered to internal tissue of the patient through the contact within a predetermined duration, and signal acquisition is performed on the internal tissue of the patient through the associated contact set to acquire the first density amplitude corresponding to the contact.

In some embodiments, the at least one processor may be configured to, when executing the computer program, acquire the second density amplitude corresponding to any contact in the following manner:

In step S401, an associated contact set of the contact is acquired. The associated contact set consists of two contacts adjacent to the contact in the circumferential direction of the segmented electrode.

In step S402, when the segmented electrode does not deliver electrical stimulation to internal tissue of the patient, signal acquisition is performed on the internal tissue of the patient through the associated contact set to acquire the second density amplitude corresponding to the contact.

In some embodiments, the at least one processor may be configured to, when executing the computer program, determine one contact among the multiple contacts as the target contact in at least one of the following manners:

A difference between the first density amplitude and the second density amplitude corresponding to each contact is acquired, and a contact corresponding to the acquired maximum difference is taken as the target contact.

For each contact and the first density amplitude and the second density amplitude corresponding to each contact, a similarity between a predicted stimulation data of each contact and historical stimulation data of the segmented electrode is acquired respectively according to the first density amplitude and the second density amplitude, the acquired similarity is taken as a first similarity, and a contact corresponding to the acquired maximum first similarity is taken as the target contact.

In some embodiments, the at least one processor may be configured to, when executing the computer program, respectively acquire the difference between the first density amplitude and the second density amplitude corresponding to each contact, and take the contact corresponding to the acquired maximum difference as the target contact in the following manner:

In step S501, a contact corresponding to a first density amplitude outside a first preset numerical range and/or a contact corresponding to a second density amplitude outside a second preset numerical range is removed.

In step S502, a difference between the first density amplitude and the second density amplitude corresponding to each contact after filtering is acquired, and the contact corresponding to the maximum difference is taken as the target contact.

In some embodiments, when the segmented electrode does not deliver electrical stimulation, for each candidate contact pair, the at least one processor may be configured to, when executing the computer program, acquire the third density amplitude corresponding to each candidate contact pair in the following manner:

In step S601, an eight-neighborhood search with the target contact as a center point is performed to acquire multiple contacts satisfying a preset distance and/or a preset positional relationship as candidate contacts, and two of the candidate contacts distributed on two sides of the target contact are taken as a candidate contact pair.

In step S602, for each candidate contact pair, the power spectral density amplitude of the bioelectrical signal corresponding to the candidate contact pair is acquired and recorded as the third density amplitude.

In some embodiments, the at least one processor may be configured to, when executing the computer program, determine one candidate contact pair from the multiple candidate contact pairs as the target acquisition contact pair corresponding to the target contact in at least one of the following manners:

A candidate contact pair corresponding to the acquired maximum third density amplitude is taken as the target acquisition contact pair.

For each candidate contact pair and the third density amplitude corresponding to each candidate contact pair, a similarity between predicted acquisition data of each candidate contact pair and historical acquisition data of the segmented electrode is acquired according to the third density amplitude, the acquired similarity is taken as a second similarity, and a contact pair corresponding to the acquired maximum second similarity is taken as the target acquisition contact pair.

In some embodiments, before step S101 is executed, the at least one processor is configured to, when executing the computer program, further execute the following steps.

User data of the patient is acquired, and the user data includes disease type information of the patient.

Frequency band parameters corresponding to any of the first density amplitude, the second density amplitude and the third density amplitude are determined according to the user data. The frequency band parameters are used to indicate the frequency band acquired during signal acquisition.

### Stimulator embodiment

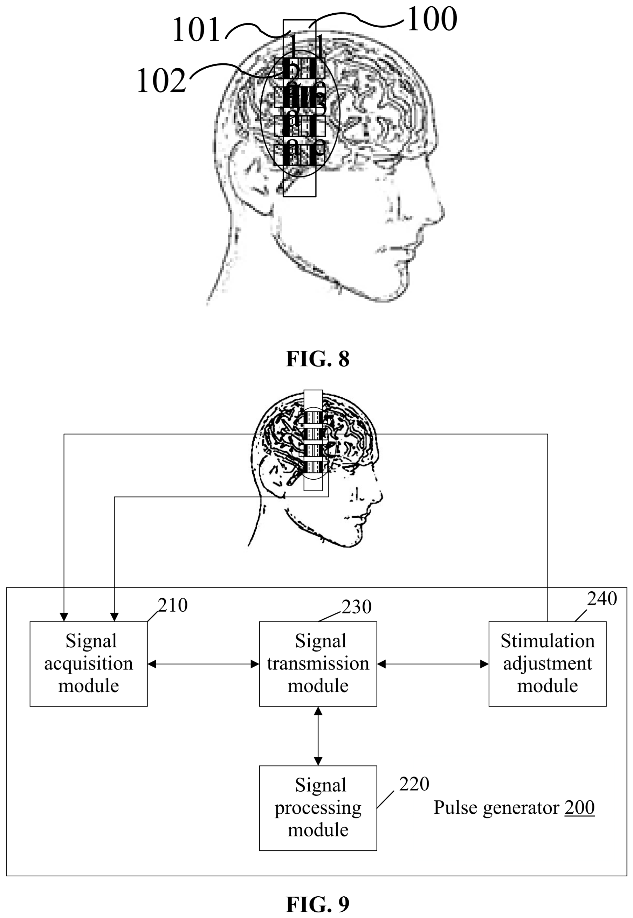

Referring to FIGS. 7 and 8, FIG. 7 is a schematic structural diagram of a stimulator according to an embodiment of the present disclosure. FIG. 8 is a schematic diagram of a segmented electrode implanted in a brain of a patient according to an embodiment of the present disclosure. The illustrated multiple contacts 102 on the segmented electrode are divided into four circumferential contact groups (consisting of contacts) spaced apart along the axial direction of the electrode lead, and each circumferential contact group includes multiple contacts 102 arranged along the circumferential direction. Each circumferential contact group is located within an oval-shaped nucleus (i.e., internal tissue) in the brain of the patient.

The embodiments of the present disclosure further provide a stimulator 20. The stimulator 20 includes a segmented electrode 100 and a pulse generator 200. The pulse generator 200 is consistent with the implementations described in pulse generator embodiment and achieves the same technical effect as the pulse generator, and some details will not be repeated here.

The segmented electrode 100 includes an electrode lead 101 and multiple contacts 102 arranged in an array along the circumferential direction of the electrode lead 101, and each contact 102 is configured to deliver electrical stimulation to the internal tissue of a patient and/or acquire a physiological signal from the internal tissue of the patient.

The pulse generator 200 (implantable pulse generator) is electrically connected to each contact 102, and the pulse generator 200 is configured to analyze the physiological signal and generate the electrical stimulation.

Referring to FIG. 9, FIG. 9 is a schematic structural diagram of a pulse generator 200 according to an embodiment of the present disclosure.

In some embodiments, the pulse generator 200 may include a signal acquisition module 210, a signal processing module 220, a signal transmission module 230, and a stimulation adjustment module 240.

The signal acquisition module 210 is configured to acquire the physiological signal through the multiple contacts 102 and amplify the acquired physiological signal.

The signal processing module 220 is configured to perform signal processing on an amplified physiological signal to acquire a power spectral density curve. The signal processing includes at least one of band-pass filtering, notch filtering and fast Fourier transform.

The signal transmission module 230 is configured to send the amplified physiological signal to the signal processing module 220.

The stimulation adjustment module 240 is configured to adjust a stimulation parameter corresponding to the electrical stimulation according to an amplitude of the physiological signal and the power spectral density curve.

Therefore, the pulse generator 200 according to the present embodiment may be used to provide the stimulation parameter suitable for different (patient) individuals to better stimulate the nervous system and acquire physiological signals. For example, since the physiological signals are typically small, the physiological signals may be amplified by the signal acquisition module 210 to facilitate processing. The amplified physiological signal is then subjected to signal processing by the signal processing module 220 to acquire the power spectral density curve. The signal processing module may perform the signal processing using methods such as band-pass filtering, notch filtering and fast Fourier transform to extract and analyze the information within a specific frequency range of the physiological signals. According to the (amplitude and power spectral density curve of) real-time acquired physiological signals, by utilizing the stimulation adjustment module 240, the stimulation parameter corresponding to the electrical stimulation can be adjusted to adapt to the physiological characteristics and responses of different individuals.

In summary, the provided pulse generator 200 can offer more personalized stimulation parameters, thus improving the stimulation effect and the accuracy of physiological signal acquisition.

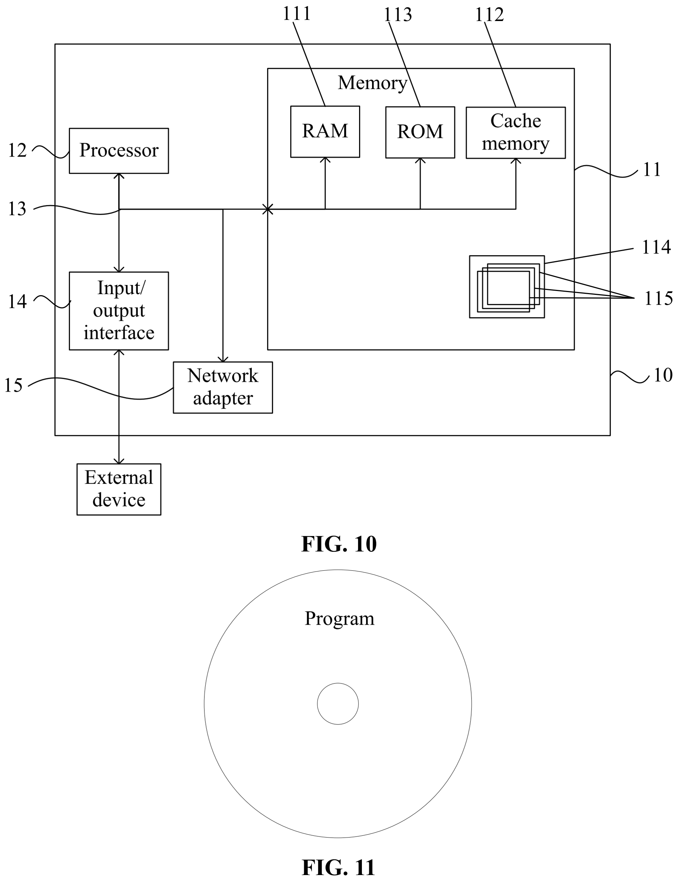

In some possible implementations, the stimulator may be an electronic device. Referring to FIG. 10, FIG. 10 is a structural block diagram of an electronic device 10 according to an embodiment of the present disclosure.

The electronic device 10 may include, for example, at least one memory 11, at least one processor 12, and a bus 13 connecting different platform systems.

The memory 11 may include a (computer-)readable medium in the form of volatile memory, such as random access memory (RAM) 111 and/or cache memory 112, and may also include read-only memory (ROM) 113.

The memory 11 also stores a computer program that can be executed by the processor 12, causing the processor 12 to implement any of the methods described above.

The memory 11 may also include a utility 114 having at least one program module 115. Such program module 115 includes an operating system, one or more application programs, other program modules and program data. Each or some combination of these examples may include an implementation of a network environment.

Correspondingly, the processor 12 can execute the computer program described above, and can execute the utility 114.

The processor 12 may employ one or more application specific integrated circuits (ASICs), digital signal processors (DSPs), programmable logic devices (PLDs), complex programmable logic devices (CPLDs), field-programmable gate arrays (FPGAs) or other electronic components.

The bus 13 may be one or more representative of several types of bus structures, including a memory bus or a memory controller, a peripheral bus, a graphics acceleration port, a processor, or a local bus using any of a variety of bus structures.

The electronic device 10 can also communicate with one or more external devices, such as a keyboard, a pointing device, a Bluetooth device, with one or more devices capable of interacting with the electronic device 10, and/or with any device (such as a router, a modem) that enables the electronic device 10 to communicate with one or more other computing devices. Such communication can be performed through the input-output interface 14. Furthermore, the electronic device 10 can also communicate with one or more networks (e.g., local area network (LAN), wide area network (WAN) and/or public network, such as the Internet) through a network adapter 15. The network adapter 15 can communicate with other modules of the electronic device 10 through the bus 13. It should be understood that, although not shown in the figure, other hardware and/or software modules may be used in combination with the electronic device 10 in practical applications, including a microcode, a device driver, a redundant processor, an external disk drive array, a redundant arrays of independent disks (RAID) system, a tape drive and a data backup storage platform, etc.

### Computer-readable storage medium embodiment

The embodiments of the present disclosure further provide a computer-readable storage medium, this embodiment is consistent with method embodiment, the same technical effect as the method can be achieved, and some details will not be repeated here.

The computer-readable storage medium stores a computer program, and when being executed by at least one processor, the computer program is configured to enable the at least one processor to implement any of the above methods or functions of any of the above electronic devices.

The computer-readable medium may be a computer-readable signal medium or a computer-readable storage medium. In the embodiments of the present disclosure, the computer-readable storage medium may be any tangible medium containing or storing a program that can be used by or in combination with an instruction execution system, apparatus or device. The computer-readable storage medium may be, for example, an electrical, magnetic, optical, electromagnetic, infrared, or semiconductor system, apparatus, or device, or any combination thereof. The computer-readable storage medium includes an electrical connection having one or more wires, a portable disk, a hard disk, a RAM, a ROM, an erasable programmable read-only memory (EPROM), a flash memory, an optical fiber, a compact disc read-only memory (CD-ROM), an optical storage device, a magnetic storage device, or any suitable combination thereof. The storage medium may be a non-transitory storage medium.

The computer-readable storage medium may include a propagated data signal with a readable program code carried therein, in a baseband or as part of a carrier wave. Such a propagated data signal may adopt a variety of forms, including an electromagnetic signal, an optical signal or any suitable combination thereof. The computer-readable storage medium may also be any computer-readable medium capable of sending, propagating, or transmitting a program for use by or in conjunction with an instruction execution system, apparatus, or device. The program code contained in the computer-readable storage medium may be transmitted by any suitable medium, including wireless, wired, optical fiber, radio frequency (RF), or any suitable combination thereof. The program code for performing the operations of the present disclosure may be written in any combination of one or more programming languages, including object-oriented programming languages such as Java, C++, as well as conventional procedural programming languages such as C or similar programming languages. The computer program may be executed entirely on a user computing device, partially executed on a user computing device, partially executed on a user computing device and partially executed on a remote computing device as an independent software package, or executed entirely on a remote computing device or server. In the case involving the remote computing device, the remote computing device may be connected to the user computing device through any type of network, including a LAN, a WAN, or may be connected to an external computing device (for example, through the Internet using an Internet service provider).

### Computer program product embodiment

The embodiments of the present disclosure further provide a computer program product, this embodiment is consistent with method embodiment, the same technical effect as the method can be achieved, and some details will not be repeated here.

The computer program product includes a computer program, and when being executed by at least one processor, the computer program is configured to enable the at least one processor to implement any of the above methods or functions of any of the above electronic devices.

Referring to FIG. 11, FIG. 11 is a schematic structural diagram of a computer program product according to an embodiment of the present disclosure.

The computer program product is used for implementing any of the above methods or implementing any of the functions of the above electronic devices. The computer program product may employ a portable CD-ROM and include a program code, and may run on a terminal device, such as a personal computer. However, the computer program product of the present disclosure is not limited thereto, and the computer program product may employ any combination of one or more computer-readable media.

## Claims

1. A pulse generator, wherein the pulse generator is configured to be implanted in a patient, the pulse generator comprises a memory and at least one processor, the memory stores a computer program, and the at least one processor is configured to, when executing the computer program, implement:
for each contact on a segmented electrode, acquiring power spectral density amplitudes of bioelectric signals corresponding to the respective contact delivering electrical stimulation to internal tissue of the patient and the respective contact not delivering electrical stimulation, respectively, and recording the power spectral density amplitudes as a first density amplitude and a second density amplitude, respectively;
determining one contact from a plurality of contacts as a target contact according to the first density amplitude and the second density amplitude corresponding to each contact;
acquiring a plurality of candidate contact pairs near the target contact according to a position of each contact on the segmented electrode;
in response to each contact not delivering electrical stimulation, acquiring a power spectral density amplitude of a bioelectric signal corresponding to each candidate contact pair among the plurality of candidate contact pairs, and recording the power spectral density amplitude as a third density amplitude; and
determining, according to a correspondence between a plurality of third density amplitudes and the plurality of candidate contact pairs, one candidate contact pair from the plurality of candidate contact pairs as a target acquisition contact pair corresponding to the target contact.

2. The pulse generator according to claim 1, wherein the at least one processor is configured to, when executing the computer program, acquire first density amplitudes and second density amplitudes corresponding to all the plurality of contacts in a following manner:
acquiring an initial contact and an acquisition sequence of density amplitudes by utilizing the position of each contact on the segmented electrode;
taking the initial contact as a starting point, sequentially acquiring the first density amplitude and the second density amplitude corresponding to each contact according to the acquisition sequence until the first density amplitudes and the second density amplitudes of all the plurality of contacts are acquired; or
taking the initial contact as a starting point, sequentially acquiring the first density amplitude of each contact according to the acquisition sequence, and after acquiring the first density amplitudes of all the plurality of contacts, acquiring the second density amplitude of each contact in the same acquisition sequence.

3. The pulse generator according to claim 2, wherein the at least one processor is configured to, when executing the computer program, acquire the first density amplitude corresponding to any contact in a following manner:
acquiring an associated contact set of the contact, wherein the associated contact set consists of two contacts adjacent to the contact in a circumferential direction of the segmented electrode; and
delivering the electrical stimulation to internal tissue of the patient through the contact within a predetermined duration, and performing signal acquisition on the internal tissue of the patient through the associated contact set to acquire the first density amplitude corresponding to the contact.

4. The pulse generator according to claim 2, wherein the at least one processor is configured to, when executing the computer program, acquire the second density amplitude corresponding to any contact in a following manner:
acquiring an associated contact set of the contact, wherein the associated contact set consists of two contacts adjacent to the contact in a circumferential direction of the segmented electrode; and
in response to the segmented electrode not delivering electrical stimulation to internal tissue of the patient, performing signal acquisition on the internal tissue of the patient through the associated contact set to acquire the second density amplitude corresponding to the contact.

5. The pulse generator according to any one of claims 1 to 4, wherein the at least one processor is configured to, when executing the computer program, determine one contact among the plurality of contacts as the target contact in at least one of following manners:
acquiring a difference between the first density amplitude and the second density amplitude corresponding to each contact, and taking a contact corresponding to an acquired maximum difference as the target contact; or
for each contact and the first density amplitude and the second density amplitude corresponding to each contact, acquiring a similarity between predicted stimulation data of each contact and historical stimulation data of the segmented electrode according to the first density amplitude and the second density amplitude, taking the acquired similarity as a first similarity, and taking a contact corresponding to an acquired maximum first similarity as the target contact.

6. The pulse generator according to claim 5, wherein the at least one processor is configured to, when executing the computer program, acquire the difference between the first density amplitude and the second density amplitude corresponding to each contact and take the contact corresponding to the acquired maximum difference as the target contact in a following manner:
removing a contact corresponding to at least one of a first density amplitude outside a first preset numerical range or a second density amplitude outside a second preset numerical range; and
acquiring a difference between the first density amplitude and the second density amplitude corresponding to each contact after filtering, and selecting the contact corresponding to the maximum difference as the target contact.

7. The pulse generator according to claim 1, wherein in response to the segmented electrode not delivering electrical stimulation, for each candidate contact pair among the plurality of candidate contact pairs, the at least one processor is configured to, when executing the computer program, acquire the third density amplitude corresponding to each candidate contact pair in a following manner:
performing an eight-neighborhood search with the target contact as a center point to acquire a plurality of contacts satisfying at least one of a preset distance or a preset positional relationship as candidate contacts, and taking two of the candidate contacts distributed on two sides of the target contact as a candidate contact pair; and
for each candidate contact pair among the plurality of candidate contact pairs, acquiring the power spectral density amplitude of the bioelectric signal corresponding to each candidate contact pair, and recording the power spectral density amplitude as the third density amplitude.

8. The pulse generator according to claim 7, wherein the at least one processor is configured to, when executing the computer program, determine the one candidate contact pair from the plurality of candidate contact pairs as the target acquisition contact pair corresponding to the target contact in at least one of following manners:
taking a candidate contact pair corresponding to an acquired maximum third density amplitude as the target acquisition contact pair; or
for each candidate contact pair among the plurality of candidate contact pairs and the third density amplitude corresponding to each candidate contact pair, acquiring a similarity between predicted acquisition data of each candidate contact pair and historical acquisition data of the segmented electrode according to the third density amplitude, taking the acquired similarity as a second similarity, and taking a contact pair corresponding to an acquired maximum second similarity as the target acquisition contact pair.

9. A stimulator, comprising:
a segmented electrode, wherein the segmented electrode comprises an electrode lead and a plurality of contacts arranged in an array along a circumferential direction of the electrode lead, and each of the plurality of contacts is configured to implement at least one of delivering electrical stimulation to internal tissue of a patient or acquiring a physiological signal from internal tissue of a patient; and
the pulse generator according to any one of claims 1 to 8, wherein the pulse generator is electrically connected to each of the plurality of contacts and configured to analyze the physiological signal and generate the electrical stimulation.

10. The stimulator according to claim 9, wherein the pulse generator comprises:
a signal acquisition module configured to acquire the physiological signal through the plurality of contacts and amplify the acquired physiological signal;
a signal processing module configured to perform signal processing on an amplified physiological signal to acquire a power spectral density curve, wherein the signal processing comprises at least one of band-pass filtering, notch filtering or fast Fourier transform;
a signal transmission module configured to send the amplified physiological signal to the signal processing module; and
a stimulation adjustment module configured to adjust a stimulation parameter corresponding to the electrical stimulation according to an amplitude of the physiological signal and the power spectral density curve.

11. A computer-readable storage medium, wherein the computer-readable storage medium stores a computer program, when being executed by at least one processor, the computer program is configured to enable the at least one processor to implement a function of the pulse generator according to any one of claims 1 to 8; or, when being executed by at least one processor, the computer program is configured to enable the at least one processor to implement a function of the stimulator according to any one of claims 9 to 10.

12. A computer program product, wherein the computer program product comprises a computer program, when being executed by at least one processor, the computer program is configured to enable the at least one processor to implement a function of the pulse generator according to any one of claims 1 to 8; or, when being executed by at least one processor, the computer program is configured to enable the at least one processor to implement a function of the stimulator according to any one of claims 9 to 10.
